# FASCICULE DE BREVET EUROPEEN

(11) **EP 0 650 331 B1**
(45) Date de publication et mention de la délivrance du brevet: **15.04.1998**
(21) Numéro de dépôt: 93915996.8
(22) Date de dépôt: 13.07.1993
(51) Int. Cl.: A23L 1/30, A23L 1/16, A23C 9/20, A23F 5/14, A21D 13/00

(54) **PRODUITS ALIMENTAIRES CONTENANT DU BENZIMIDAZOLE**
BENZIMIDAZOLE ENTHALTENE NAHRUNGSMITTEL
FOOD PRODUCTS CONTAINING BENZIMIDAZOLE

(30) Priorité: 15.07.1992 FR 9208743
(43) Date de publication de la demande: 03.05.1995
(73) Titulaire: TYORTYALIAN, Carlos, F-91440 Bures-sur-Yvette (FR)
(72) Inventeur: TYORTYALIAN, Carlos, F-91440 Bures-sur-Yvette (FR)
(74) Mandataire: Portal, Gérard
(86) Numéro de dépôt international: FR9300716
(87) Numéro de publication internationale: WO9402035

(56) Documents cités:
- FR-A- 1 473 828
- FR-A- 2 260 995
- US-A- 4 056 108
- Food Science & Technology Abstracts, 1985. Acc. No.85-06-c0023, International Food Information Service; F.W. van der Kreek: "Animal Drugs and the Evaluation of Residues in Milk and Meat"

## Description

La présente invention a pour objets des produits alimentaires et leurs procédés de préparation ou de traitement. Lesdits produits alimentaires contiennent une substance chimique qui facilite leur assimilation par l'organisme.

La présente invention apporte, plus précisément une solution au problème de l'assimilation des aliments à caractère acide, connus pour exciter les sécrétions gastriques et duodénales.

L'assimilation de ces aliments entraîne, occasionnellement, chez les individus bien portants, des malaises (mauvaise digestion, brûlures d'estomac...) à caractère bénins. Elle peut, chez d'autres individus, générer à la longue des affections plus graves, susceptibles de déboucher sur un ulcère ou une péritonite.

Les traitements classiques, pour ce type de maux, consistent à supprimer du régime alimentaire tout aliment excitant les sécrétions gastriques et duodénales et/ou à administrer :
- des médicaments destinés à neutraliser l'acidité gastrique, tel que le bicarbonate de soude ; et/ou
- des médicaments destinés à protéger de l'ulcération, tel que le carbonate de bismuth ; et/ou
- des médicaments destinés à diminuer l'hypersécrétion, tels que l'atromine et la belladone.

Ces derniers médicaments ont des effets secondaires gênants, tout comme l'histamine et l'extrait parathyroïdien parfois employés pour calmer les douleurs.

Ces traitements classiques sont contraignants et mettent parfois en oeuvre des médicaments coûteux, difficiles à administrer, voire même dangereux à longue échéance.

Il conviendrait de substituer auxdits médicaments, pour tout ou partie au moins, un produit, peu cher, inoffensif et facile à administrer.

On propose selon la présente invention, de faire intervenir un tel produit : le benzimidazole et/ou au moins un de ses dérivés, en mélange direct avec les aliments.

Ledit benzimidazole répond à la formule :

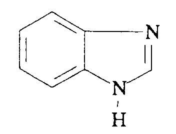

Ses dérivés, par addition ou substitution, répondent avantageusement à la formule ci-après : 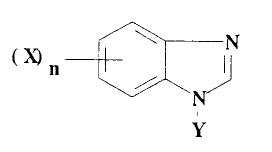 dans laquelle
- X et Y représentent, indépendamment un groupe alkyle notamment méthyle, un groupe phényle, un halogène...
- n = 1 à 4.

Le benzimidazole est un composé chimique bien connu, dont la première synthèse remonte à plus d'un siècle (synthèse par réaction de l'ophénylénediamine et de l'acide formique : Wundt, Ber. **11**,826(1878)).

Ce composé est très stable notamment en température et il résiste à l'action des acides, notamment sulfurique et chlorhydrique, concentrés et des alcalis.

Ce composé possède par ailleurs une activité biologique bien connue, en raison de sa parenté chimique avec l'histamine, et de sa présence dans la molécule de la vitamine B12 (où il forme un pont entre le cobalt et l'une des chaînes latérales, ladite chaîne se raccrochant par un résidu amino-alcool).

Le benzimidazole et ses dérivés, sont également déjà connus pour présenter des propriétés pharmacologiques, qui ne sont pas incompatibles avec leur emploi dans le cadre de la présente invention.

Il a été décrit par le document FR-A-2 26O 995 l'emploi de benzimidazole et ses dérivés, comme médicamet pour le traitement des affections gastro-duodénales.

Par ailleurs, le document US-A-4 O56 1O8 est relatif à un produit de tabac ou des aliments contenant de l'imidazole ou du benzimidazole pour améliorer le goût et/ou l'arôme de ces produits.

On a notamment décrit l'action de certains desdits dérivés sur la vasomotricité, l'action de certains autres contre la malaria.

On a également montré que certains desdits dérivés possèdent des propriétés antifongiques remarquables, de spectre supérieur à celui de la griséofulvine, dues au blocage de la synthèse protéique mais pouvant être inhibées par l'action antagoniste de l'adénine, de la guanine, des acides nucléiques et de la vitamine B12, voir en particulier le document FR-A-1,4 73,828;

L'étude des diverses propriétés pharmacologiques du benzimidazole et de ses dérivés a permis de mettre en évidence l'innocuité de ces substances. La dose léthale à 50 % (DL 50) du benzimidazole a été notamment déterminée par la méthode dite "Up and Down" chez la souris. Ladite dose léthale varie après 30 jours, de 0,520 à 0,610 mg/g.

Dans le cadre de ses travaux, le Demandeur a mis en évidence l'affinité sélective et la concentration persistante du benzimidazole et de ses dérivés, au niveau des muqueuses gastro-duodénales et l'intérêt qu'il y a à ingérer le(s)dit(s) produit(s) en même temps que des produits alimentaires, susceptibles de provoquer stimulation et hypersécrétion desdites muqueuses.

L'affinité sélective du benzimidazole pour la muqueuse gastrique, chez la souris et le chien comme chez l'homme, a été démontrée par des techniques autoradiographiques et gamma-scintigraphiques, utilisant du benzimidazole marqué au carbone 14 ou à l'iode 131 et injecté par voie intrapéritonéale ou intraveineuse.

Dans les deux cas, il a été constaté que la répartition du produit marqué est homogène dès 15 minutes après l'injection, mais qu'après 3 heures, la radioactivité de la plupart des tissus est très basse, sauf celle de l'estomac et du contenu gastrique qui continue à croître et se maintient jusqu'au-delà de 6 heures pour disparaître dans les 24 heures.

Ces tests ont par ailleurs montré que le benzimidazole s'élimine facilement et totalement, surtout par voie urinaire, et est exempt de toute toxicité rémanente chez l'homme comme chez l'animal, même aux concentrations observées au niveau des muqueuses gastro-duodénales.

Le Demandeur a également mesuré la DE 50, sur des souris (22-28 g), dans des tests d'anti-salivation (salivation induite par injection sous-cutanée de pilo-carpine : DE₅₀ > 100 mg/kg p.o) et des tests de motricité intestinale (en utilisant du charbon : DE₅₀ > 100 mg/kg p.o).

Le benzimidazole (jusqu'à des doses de plus de 100 mg/kg p.o) n'a pas d'influence sur la salivation et ne réduit pas la motricité intestinale.

On préconise, selon la présente invention, l'intervention de ce composé et/ou d'au moins un de ses dérivés dans des produits alimentaires.

La présente invention a donc pour objet l'utilisation du benzimidazole et/ou au moins un de ses dérivés comme agent facilitant l'assimilation par l'organisme d'un produit alimentaire à caractère acide qui, ingéré, est susceptible de provoquer stimulation et hypersécrétion de la muqueuse gastrique et/ou de la muqueuse duodénale.

Les produits alimentaires contiennent au sens de l'invention ledit benzimidazole et/ou au moins un de ses dérivés, en une quantité efficace pour l'obtention du résultat recherché : meilleure assimilation par l'organisme.

On aboutit ainsi, de façon originale, à une parfaite digestion desdits aliments, que l'on a, selon l'invention, rendus digestibles.

La combinaison : produit alimentaire/benzimidazole (et/ou au moins un de ses dérivés), selon l'invention est très avantageuse. Elle permet d'avorter toute affection gastro-duodénale (non tumorale), d'éviter toute apparition de douleurs (maux d'estomac), après l'ingestion de produits, réputés lourds à digérer. Elle a un effet préventif.

Le Demandeur a, par ailleurs, mis en évidence, le fait que les affections gastriques touchaient également les enfants et les nouveaux-nés. Ainsi, selon un de ses aspects, l'invention a pour objet des produits alimentaires pour enfants et/ou nouveaux-nés, qui contiennent du benzimidazole et/ou au moins un de ses dérivés. On citera notamment le lait, éventuellement en poudre, pour nourrissons.

Des produits alimentaires, auxquels on associe avantageusement selon l'invention du benzimidazole et/ou un de ses dérivés, sont listés ci-après : pâtes alimentaires, pain, sauces, confiture, marmelade, gelées, sucreries, glaces, chocolats, entremets, biscuits, café, thé, saucisses, huiles...

Cette liste est évidemment non exhaustive.

On précisera notamment ici que dans le présent texte le terme "produit alimentaire" englobe les boissons telles que les boissons alcoolisées, les jus de fruit, les eaux minérales gazeuses ou non ...

La quantité de benzimidazole et/ou analogue(s), qui intervient, n'est pas critique dans la mesure où le produit n'est pas toxique et ne présente pas d'effet secondaire. On estime à environ 15 à 30 mg la quantité journalière qu'un adulte peut ingérer (à 5 à 15 mg celle qu'un nouveau-né ou enfant (8-10 ans) peut ingérer) sans aucun danger.

Il convient évidemment de mettre suffisamment de produit pour obtenir le résultat escompté. On notera incidemment que l'intervention du benzimidazole (et/ou d'au moins un de ses dérivés) au sein des aliments, n'altère ni leur flaveur (goût et odeur), ni leur aspect.

L'invention a notamment pour objet :
- du pain contenant, pour 100 g, de 15 à 25 mg de benzimidazole et/ou d'au moins un de ses dérivés ;
- du café contenant, pour 1 kg, environ 500 mg de benzimidazole et/ou d'au moins un de ses dérivés ;
- des pâtes alimentaires contenant, pour 1 kg, environ 150 mg de benzimidazole et/ou d'au moins un de ses dérivés ;
- des sauces contenant, pour 100 g, de 50 à 60 mg de benzimidazole et/ou d'au moins un de ses dérivés ;
- de la confiture contenant, pour 1 kg, environ 100 mg de benzimidazole et/ou d'au moins un de ses dérivés ;
- des boissons alcoolisées telles que whisky, cognac, liqueurs, vins, champagnes... contenant, par exemple, 60 à 200 mg/litre de benzimidazole et/ou d'au moins un de ses dérivés ;
- des bières contenant par exemple de 30 à 70 mg/litre de benzimidazole et/ou d'au moins un de ses dérivés ;
- des jus de fruits ou analogues, tels que jus de raisin, d'orange, de pamplemousse, de citron, d'ananas, d'abricots, de pêche, de pomme ou limonade ou coca-cola ou cidre... contenant par exemple de 25 à 60 mg/litre de benzimidazole et/ou d'au moins un de ses dérivés ;
- des eaux minérales gazeuses ou non... contenant par exemple de 10 à 25 mg/litre de benzimidazole et/ou d'au moins un de ses dérivés.

Pour la fabrication de pain qui inclut, selon l'invention, du benzimidazole et/ou au moins un de ses dérivés, on utilisera avantageusement de la farine qui contient de 20 à 25 mg dudit benzimidazole et/ou au moins un de ses dits dérivés par kg.

Il ressort clairement des propos ci-dessus, que l'intervention, selon l'invention, du benzimidazole et/ou d'au moins un de ses dérivés n'est pas strictement limitée à telle ou telle quantité pour tel ou tel type de produit.

La combinaison produit alimentaire/benzimidazole (et/ou au moins un de ses dérivés) ne pose, par ailleurs, aucun problème d'élaboration ; compte tenu notamment de la stabilité desdites substances chimiques.

Selon un autre de ses aspects, l'invention a pour objets un procédé pour la préparation d'un produit alimentaire et un procédé de traitement d'un produit alimentaire à caractère acide qui, ingéré, est susceptible de provoquer stimulation et hypersécrétion de la muqueuse gastrique et/ou de la muqueuse duodénale, pour faciliter son assimilation par l'organisme ; procédés qui consistent :
- soit à inclure du benzimidazole et/ou au moins un de ses dérivés, au cours de l'élaboration dudit produit alimentaire ;
- soit à ajouter ledit benzimidazole et/ou au moins un de ses dérivés dans ledit produit alimentaire élaboré.

On ne peut exclure l'intervention dudit benzimidazole et/ou d'au moins un de ses dérivés en cours d'élaboration du produit et au stade du produit final.

On propose donc, selon la présente invention, une utilisation originale du benzimidazole et de ses dérivés ; utilisation au sein de produits alimentaires pour en faciliter l'assimilation par l'organisme. En d'autres termes, on propose selon l'invention, une méthode pour faciliter l'assimilation de produits alimentaires par l'organisme ; méthode qui consiste à incorporer en leur sein du benzimidazole et/ou au moins un de ses dérivés.

## Revendications

1. Utilisation du benzimidazole et/ou au moins un de ses dérivés comme agent facilitant l'assimilation par l'organisme d'un produit alimentaire à caractère acide qui, ingéré, est susceptible de provoquer stimulation et hypersécrétion de la muqueuse gastrique et/ou de la muqueuse duodénale.

2. Utilisation selon la revendication 1, caractérisée en ce que le dérivé de benzimidazole précité répond à la formule chimique suivante : 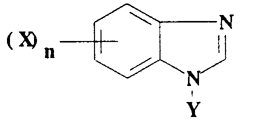 dans laquelle
- X et Y représentent, indépendamment un groupe alkyle notamment méthyle, un groupe phényle, un halogène ; et
- n = 1 à 4.

3. Utilisation selon la revendication 1 ou 2, caractérisée en ce que le produit alimentaire précité à caractère acide entre dans l'alimentation pour nouveaux-nés ou enfants.

4. Utilisation selon la revendication 3, caractérisée en ce que le produit alimentaire à caractère acide précité consiste en du lait, éventuellement en poudre, pour nourrissons.

5. Utilisation selon la revendication 1 ou 2, caractérisée en ce que le produit alimentaire précité à caractère acide est choisi parmi les pâtes alimentaires, le pain, les sauces, la confiture, la marmelade, les gelées, les sucreries, les glaces, les chocolats, les entremets, les biscuits, le café, le thé, les saucisses, les huiles, une boisson telle qu'une boisson alcoolisée, un jus de fruits, une eau minérale gazeuse ou non, la bière.

6. Utilisation selon l'une des revendications 1, 2; 3, 5, caractérisée en ce que le produit alimentaire précité est du pain et en ce qu'il contient, pour 100g, de 15 à 25 mg de benzimidazole et/ou d'au moins un de ses dérivés précités.

7. Utilisation selon l'une des revendications 1 à 3, 5, caractérisée en ce que le produit alimentaire précité est du café et en ce qu'il contient, pour 1 kg, environ 500 mg de benzimidazole et/ou d'au moins un de ses dérivés précités.

8. Utilisation selon l'une des revendications 1 à 3, 5, caractérisée en ce que le produit alimentaire précité est une pâte alimentaire, et en ce qu'elle contient pour 1 kg, environ 150 mg de benzimidazole et/ou d'au moins un de ses dérivés précités.

9. Utilisation selon l'une des revendications 1 à 3, 5, caractérisée en ce que le produit alimentaire précité est une sauce, et en ce qu'elle contient, pour 100 g, de 50 à 60 mg de benzimidazole et/ou d'au moins un de ses dérivés précités.

10. Utilisation selon l'une des revendications 1 à 3, 5, caractérisée en ce que le produit alimentaire précité est de la confiture et en ce qu'elle contient, pour 1 kg, environ 100 mg de benzimidazole et/ou au moins un de ses dérivés précités.

11. Procédé de traitement d'un produit alimentaire à caractère acide qui, ingéré, est susceptible de provoquer stimulation et hypersécrétion de la muqueuse gastrique et/ou de la muqueuse duodénale, pour faciliter son assimilation par l'organisme, consistant à inclure en cours d'élaboration dudit produit et/ou à rajouter audit produit élaboré, une quantité efficace de benzimidazole et/ou d'au moins un de ses dérivés, tel que défini à l'une quelconque des revendications 2 à 1O.

## Claims

1. Use of benzimidazole and/or at least one of its derivatives as an agent promoting assimilation by the organism of a food product having an acid character which, when ingested is liable to cause stimulation and hypersecretion of the gastric and/or duodenum mucosae.

2. Use according to claim 1, characterized in that the aforesaid benzimidazole derivative has the following chemical formula: 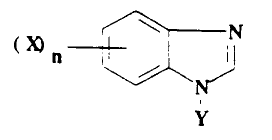 in which
- X and Y independently represent an alkyl group, notably methyl, a phenyl group or an halogen atom; and
- n = 1 to 4.

3. Use according to claim 1 or 2, characterized in that the aforesaid food product having an acid character is contained in food products for children or newborn babies.

4. Use according to claim 3, characterized in that the aforesaid food product having an acid character consists in milk, in powder form if appropriate, for infants.

5. Use according to claim 1 or 2, characterized in that the aforesaid food product having an acid character is selected from pasta, bread, sauces, jam, marmelade, jellies, sweets, icecreams, chocolate, desserts, biscuits, coffee, tea, sausages, oils, a drink such as an alcoholic drink, a fruit juice, a gaseous or non gaseous mineral water, beer.

6. Use according to one of claims 1, 2, 3, 5, characterized in that the aforesaid food product is bread and in that it contains, per 100 grams, from 15 to 25 mg of benzimidazole and/or at least one of its aforesaid derivatives.

7. Use according to one of claims 1 to 3, 5, characterized in that the aforesaid food product is coffee and in that it contains, per 1 kilogram, about 500 mg of benzimidazole and/or at least one of its aforesaid derivatives.

8. Use according to one of claims 1 to 3, 5, characterized in that the aforesaid food product is a pasta and in that it contains, per 1 kilogram, about 150 mg of benzimidazole and/or at least one of its aforesaid derivatives.

9. Use according to one of claims 1 to 3, 5, characterized in that the aforesaid food product is a sauce and in that it contains, per 100 g, from 50 to 60 mg of benzimidazole and/or at least one of its aforesaid derivatives.

10. Use according to one of claims 1 to 3, 5, characterized in that the aforesaid food product is a jam and in that it contains, per 1 kilogram, about 100 mg of benzimidazole and/or at least one of its aforesaid derivatives.

11. Method for treating a food product having an acid character which, when ingested is liable to cause stimulation and hypersecretion of the gastric and/or duodenum mucosae, for promoting its assimilation by the organism, said method consisting in including during the preparation of said product and/or in adding to said prepared product, an efficient amount of benzimidazole and/or at least one of its derivatives, as defined in any one of claims 2 to 10.

## Patentansprüche

1. Verwendung von Benzimidazol und/oder zumindest einem seiner Derivate als Mittel zur Erleichterung der Assimilation eines Nahrungsmittelprodukts mit Säurecharakter, das nach der Aufnahme eine Stimulation und Hypersekretion der Magenschleimhaut und/oder der Zwölffingerdarmschleimhaut hervorrufen kann, durch den Organismus.

2. Verwendung nach Anspruch 1, dadurch gekennzeichnet, daß das Benzimidazol-Derivat die folgende chemische Formel aufweist: 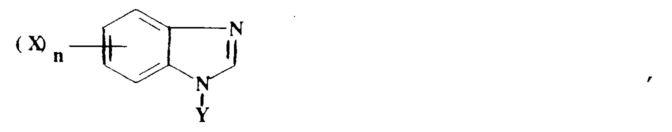 worin X und Y unabhängig eine Alkyl-Gruppe, insbesondere Methyl, eine Phenyl-Gruppe, ein Halogen bedeuten; und n = 1 bis 4.

3. Verwendung nach Anspruch 1 oder 2, dadurch gekennzeichnet, daß das vorerwähnte Nahrungsmittelprodukt mit Säurecharakter in Nahrung für Neugeborene oder Kinder enthalten ist.

4. Verwendung nach Anspruch 3, dadurch gekennzeichnet, daß das vorerwähnte Nahrungsmittelprodukt mit Säurecharakter aus Milch, gegebenenfalls in Pulverform, für Säuglinge besteht.

5. Verwendung nach Anspruch 1 oder 2, dadurch gekennzeichnet, daß das vorerwähnte Nahrungsmittelprodukt mit Säurecharakter ausgewählt ist aus Nahrungsmittelbrei, Brot, Saucen, Konfitüre, Marmelade, Gelees, Süßigkeiten, Speiseeis, Schokolade, Süßspeisen, Biskuit, Kaffee, Tee, Wurst, Öl, Getränken, wie alkoholischen Getränken, Fruchtsaft, kohlensäurehaltigem oder -freiem Mineralwasser, Bier.

6. Verwendung nach einem der Ansprüche 1, 2, 3 und 5, dadurch gekennzeichnet, daß das vorerwähnte Nahrungsmittelprodukt Brot ist, und daß es pro 100 g 15 bis 25 mg Benzimidazol und/oder zumindest eines seiner Derivate enthält.

7. Verwendung nach einem der Ansprüche 1 bis 3 und 5, dadurch gekennzeichnet, daß das vorerwähnte Nahrungsmittelprodukt Kaffee ist, und daß er pro 1 kg etwa 500 mg Benzimidazol und/oder zumindest eines seiner Derivate enthält.

8. Verwendung nach einem der Ansprüche 1 bis 3 und 5, dadurch gekennzeichnet, daß das vorerwähnte Nahrungsmittelprodukt ein Nahrungsmittelbrei ist, und daß er pro 1 kg etwa 150 mg Benzimidazol und/oder zumindest eines seiner Derivate enthält.

9. Verwendung nach einem der Ansprüche 1 bis 3 und 5, dadurch gekennzeichnet, daß das vorerwähnte Nahrungsmittelprodukt eine Sauce ist, und daß sie pro 100 g 50 bis 60 mg Benzimidazol und/oder zumindest eines seiner Derivate enthält.

10. Verwendung nach einem der Ansprüche 1 bis 3 und 5, dadurch gekennzeichnet, daß das vorerwähnte Nahrungsmittelprodukt Konfitüre ist, und daß sie pro 1 kg etwa 100 mg Benzimidazol und/oder zumindest eines seiner Derivate enthält.

11. Verfahren zur Behandlung eines Nahrungsmittelprodukts mit Säurecharakter, das nach der Aufnahme eine Stimulation und Hypersekretion der Magenschleimhaut und/oder der Zwölffingerdarmschleimhaut hervorrufen kann, zur Erleichterung seiner Assimilation durch den Organismus, welches darin besteht, daß eine wirksame Menge von Benzimidazol und/oder zumindest einem seiner Derivate, wie in einem der Ansprüche 2 bis 10 definiert, während der Verarbeitung des Produkts eingeschlossen und/oder dem verarbeiteten Produkt zugesetzt wird.
